# EUROPEAN PATENT SPECIFICATION

(11) **EP 4 195 996 B1**
(45) Date of publication and mention of the grant of the patent: **18.03.2026**
(21) Application number: 21885544.3
(22) Date of filing: 26.10.2021
(51) Int. Cl.: A61B 1/005, A61B 1/05, A61B 1/313, A61B 1/00, A61B 17/34

(54) **PORTABLE DISPOSABLE MICROPERITONEOSCOPE FOR DIRECT VISUALISATION OF PERITONEAL CAVITY OF A PATIENT AT THE POINT-OF-CARE**
TRAGBARES EINWEG-MIKROPERITONEOSKOP ZUR DIREKTEN VISUALISIERUNG DER PERITONEALHÖHLE EINES PATIENTEN AM VERSORGUNGSORT
MICROPÉRITONÉOSCOPE JETABLE PORTABLE POUR LA VISUALISATION DIRECTE DE LA CAVITÉ PÉRITONÉALE D'UN PATIENT AU NIVEAU DU POINT DE SOIN

(30) Priority: 26.10.2020 IN 202041046689
(43) Date of publication of application: 21.06.2023
(73) Proprietor: Patil, Adarsh Malagouda, Bangalore 560017 (IN)
(72) Inventor: GADAGKAR, Hrishikesh, Chadds Ford. USA, Pennsylvania 19317 (US); PATIL, Adarsh Malagouda, Bangalore North, Bangalore 560017 (IN)
(74) Representative: ip21 Ltd
(86) International application number: PCT/IN2021/051016
(87) International publication number: WO 2022/091124

(56) References cited:
- CN-A- 105 726 075
- CN-B- 105 726 075
- CN-U- 205 197 954
- DE-A1- 102010 051 502
- US-A1- 2010 249 496
- US-A1- 2019 365 415

## Description

### BACKGROUND

### Technical Field

The embodiments herein generally relate to an apparatus for internal inspection of the peritoneal cavity and more particularly, to a portable and disposable MicroPeritoneoscope and accessories for direct visualization of the peritoneal cavity of a patient at the Point-of-Care (POC) such as hospital, private clinics, physician's office, intensive care unit, temporary or field patient care facilities.

### Description of the related art

Physicians rely on a wide variety of diagnostic imaging modalities such as Ultrasound, CT, MRI, and PET to aid in diagnosis and to monitor the progress of treatments. However, many times they fail to assist in being able to provide an exact clinical diagnosis. For example, TB of the abdomen, peritoneal secondaries, post-operative adhesions, endometriosis, abdominal cancer, etc cannot be diagnosed with these tests and require direct visualization. For direct visual examination of the peritoneal cavity, the patient has to undergo a diagnostic laparotomy or diagnostic laparoscopy under general anesthesia. A laparotomy requires a large incision for direct visualization of the organs. Diagnostic Laparoscopy involves the examination of the peritoneal cavity and some of its contained organs using light and endoscope similar to those of a cystoscope. Access is gained using a trocar pushed through the abdominal cavity into the peritoneal cavity avoiding injuring the internal organs like the intestine and major vessels like the aorta or inferior vena cava. The peritoneal cavity is inflated using gas to create space for visualization. These procedures are not only a major psychological burden for the patient but also expensive and have significant morbidity associated with them. Further, there is always a risk of additional complications related to anesthesia and the procedure itself like injury to internal organs during the procedure and post operative wound infection etc. Operating rooms & laparoscopic equipment and diagnostic tools like CT, MRI & PET require significant investment, maintenance and are generally available only in major cities and secondary and tertiary hospitals.

The existing laparoscopic procedures are unsuitable for remote areas or where diagnostic imaging facilities and well-equipped operation rooms are not available, for example, at military field hospitals, and trauma centers, standalone Intensive Care Unit.

Patient who are candidates for a major surgery require adequate pre-surgery assessment and planning, without which the surgery often leads to suboptimal clinical outcomes. Currently available pre-surgical evaluation modalities such as USG, CT, MRI, PET scans often do not provide accurate clinical assessment.

Often post-operatively, patients develop complications like bleeding, Anastomotic leak, graft failure, etc. which are either not picked up accurately with the existing diagnostic imaging tools such as CT, MRI, or USG or the patients are too unstable hemodynamically and have to be transported to undergo diagnostic imaging tests.

It is fairly common for patients undergoing chemotherapy to require monitoring to access the response to the chemotherapy.

In addition, patients who are under critical care with ventilator support have restricted mobility and are also hemodynamically unstable. In case of certain diseases like infection or infarction of the intestine or blunt trauma to the abdomen, etc. It is impractical to transport these patients from intensive care unit for diagnostic evaluation. Patients require visual assessment of their peritoneal cavity for better disease management. At present, it is not feasible to visually inspect the peritoneal cavity by the bedside. Laparotomy cannot be carried out by the bedside and laparoscopy procedures require large insufflation of CO₂ and require larger and/or multiple ports for access. Laparoscopy equipment is generally bulky with a large screen, requires an insufflator connected to a large CO₂ cylinder, and has a bulky sophisticated video processing unit and an insufflator. Laparoscopes have to be cleaned and sterilized after every procedure and thus have a longer turnaround time and pose a risk for cross-contamination leading to infections. This makes the procedure unsuitable to be conducted under local anesthesia and the procedure needs to be carried out under general anesthesia in an operating room at a secondary or tertiary level hospital.

*Min Su Kim et al* disclose a percutaneous ultrathin flexible endoscope (PUFP) to access the peritoneum percutaneously under general anesthesia. US20050165276A1 relates to an endoscopic device including a steerable distal portion and an automatically controlled proximal portion which may be controlled by a physician or surgeon to facilitate steering the device while the proximal portion may be automatically controlled by, e.g., a controller or computer. US5483951A discloses an endoscopic sheath assembly that has a thin-walled flexible endoscope tube that is adapted to fit over, tightly surround and isolate at least a portion of an elongated, flexible endoscope used during therapeutic or diagnostic endoscopic procedures. The endoscope articulates about a neutral bending plane. US5176649A discloses an insertion device and a flexible sheath for inserting an endoscope into a body cavity through an abdominal wall. Once inserted, the insertion device is withdrawn from the sheath. Following withdrawal, the sheath is secured in the body wall by its adhesive collar and enables the multiple passages of an endoscope or an endoscopic operating instrument during the endoscopic procedure. US20100249496A1 describes a rigid optical scope designed primarily for surgical interventions in a fully equipped operating room under general anesthesia. Its inclusion of lateral working channels and a central channel for surgical instruments increases the device's size and complexity, making it less suitable for minimally invasive diagnostic applications in emergency or outpatient settings. The rigid nature of the optical scope in US20100249496A1 limits maneuverability, restricting its ability to navigate confined anatomical regions such as the pelvis or the diaphragmatic surface of the liver. CN105726075A describes a balloon-based insufflation mechanism that is specifically designed for retroperitoneal procedures, limiting its applicability to broader diagnostic applications within the peritoneal cavity. The reliance on a balloon for tissue separation restricts flexibility and adaptability, as it is not suitable for direct peritoneal cavity insufflation. Additionally, the use of a manual insufflator bulb in CN105726075A is primarily focused on mechanically expanding the retroperitoneal space, which does not provide the same level of control or comfort required for peritoneal diagnostics under local anesthesia. The balloon mechanism lacks the ability to gradually and precisely adjust intra-abdominal pressure in response to patient feedback, a critical factor in procedures performed while the patient is awake.

In light of the foregoing discussion, there arises a need for a low-cost disposable, fully self-contained scope with a small diameter that would be highly desirable for a physician to perform direct visual examination of peritoneal cavity at point of care, overcoming much of the above-mentioned drawbacks of the conventional laparoscopy technology and provide an alternative over the use of diagnostic laparotomy and diagnostic laparoscopy.

### OBJECT OF THE INVENTION

An object of the present invention is to develop a disposable, portable handheld MicroPeritoneoscope with integrated camera, screen, processor, battery, and biopsy forceps to perform peritoneoscopy under local anesthesia or local anesthesia with sedation at point of care. It has low acquisition costs. As the scope is disposable it does not require reprocessing thus saves cost, eliminates turnaround time and reduces the risk associated with cross-contamination.

Another object of the invention is to provide a MicroPeritoneoscope with a small diameter which reduces pain with minimal tissue damage and enables peritoneoscopy to be performed as an outpatient procedure which is cost-effective and safer with minimal discomfort to the patient.

Another object of this invention is to provide a peritoneoscopy port which provides an opportunity for bedside sequential monitoring of intra-abdominal conditions of patients with post-operative complications or patients undergoing chemotherapy to access the response to the chemotherapy.

Another object of the present invention is to provide a portable hand-held MicroPeritoneoscope suitable for remote areas where an imaging facility is not available, for example, military field hospitals, trauma centers, and standalone Intensive care units.

Another object of the present invention is to provide an outpatient-based pre-planning procedure or a staging procedure in patients who are candidates for major or supra major surgery.

Another object of the present invention is to provide an opportunity to perform bedside peritoneoscopy in patients who are unfit for general anesthesia because of comorbid conditions like lung infection or patients in intensive care units with ventilator support who are immobile and hemodynamically unstable because of certain diseases like, infection or infarction of intestine or blunt trauma abdomen.

A further object of the present invention is to provide a complete disposable kit that would facilitate peritoneal cavity examination at the point of care.

### STATEMENT OF INVENTION

The invention is defined by the independent claim. The dependent claims define advantageous embodiments.

A portable and disposable MicroPeritoneoscope for direct visualization of the peritoneal cavity by entering from abdominal wall of a patient with visual guidance under local anesthesia at point of care, the portable and disposable MicroPeritoneoscope comprises:
an optical scope comprising a proximal portion and a distal portion, the proximal portion is attached to a handpiece facilitating advancement and retraction of the optical scope, the proximal portion comprises a biopsy instrument entry port to provide access into the peritoneal cavity, the distal portion comprises a camera, a biopsy exit port, and at least one light source, the distal portion of the optical scope is flexible forming a flexible distal portion with a radius of curvature ranging from 0 degrees to 140 degrees, the distal portion of the optical scope is controlled by manipulators for visualization the peritoneal cavity from multiple angles;
a tubular optical trocar comprising an optical port inner sheath and a peritoneoscopy port, the optical port inner sheath comprises an opening configured to receive the optical scope and a glass tip to separate muscle layers in the peritoneal cavity under vision in the abdominal wall, the peritoneoscopy port comprises an opening to receive the optical port inner sheath and a with an eye to fix with skin of the patient, the optical port inner sheath is configured to pass through the peritoneoscopy port and fixed inside the peritoneoscopy port;
a manual insufflator bulb for insufflation of atmospheric air with a one-way valve to expand the space within the peritoneal cavity facilitating the optical scope to move within the peritoneal cavity for visualization, the manual insufflator bulb is connected to the peritoneoscopy port;
a digital display that is connected to the camera of the optical scope for visualizing the peritoneal cavity, the digital display is attached to the handpiece; and
a biopsy instrument that is introduced through a biopsy instrument entry port for obtaining a biopsy sample from intraperitoneal structures, the distal end of the biopsy instrument passes through the biopsy exit port of the optical scope to access organs or soft tissue within the peritoneal cavity for obtaining the biopsy sample.

### SUMMARY OF THE INVENTION

In view of the foregoing, an embodiment herein provides a portable and disposable MicroPeritoneoscope for direct visualization of the peritoneal cavity by entering from the abdominal wall of a patient with visual guidance under local anesthesia at point of care. The portable and disposable MicroPeritoneoscope includes an optical scope comprising a proximal portion and a distal portion. The proximal portion is attached to a handpiece facilitating advancement and retraction of the optical scope. The proximal portion includes a biopsy instrument entry port to provide access into the peritoneal cavity. The distal portion includes a camera, a biopsy exit port, and at least one light source. The distal portion of the optical scope is flexible forming a flexible distal portion with a radius of curvature ranging from 0 degrees to 140 degrees. The distal portion of the optical scope is controlled by manipulators for visualization the peritoneal cavity from multiple angles. A tubular optical trocar includes an optical port inner sheath and a peritoneoscopy port. The optical port inner sheath includes an opening configured to receive the optical scope and a glass tip to separate muscle layers under vision in the abdominal wall and provide a safe entry into the peritoneal cavity without injuring the internal organs. The peritoneoscopy port includes an opening to receive the optical port inner sheath and an eye feature to fix with skin of the patient. The optical port inner sheath is configured to pass through the peritoneoscopy port. A manual insufflator bulb for insufflation of atmospheric air with a one-way valve to expand the space within the peritoneal cavity facilitates the optical scope to move within the peritoneal cavity for visualization. The insufflator bulb is connected to the peritoneoscopy port. A digital display is connected to the camera of the optical scope for visualizing the peritoneal cavity. The digital display is attached to the handpiece. The digital display is permanently attached to the handpiece. In some embodiments, the digital display is temporarily attached to the handpiece. A biopsy instrument is introduced through the biopsy instrument entry port for obtaining a biopsy sample from intraperitoneal structures. The distal end of the biopsy instrument passes through the biopsy exit port of the optical scope to access the organ of soft tissue within the peritoneal cavity for obtaining the biopsy sample.

**In** some embodiments, the optical scope is of length that ranges from 10 cm to 45 cm and a breadth that ranges from 1 mm to 10 mm.

**In** some embodiments, a probe is inserted through the biopsy instrument entry port of the optical scope to assess the density and vascularity of intra-peritoneal structures or to ablate and cauterize a blood vessel. The probe is selected from an ultrasound probe, a laser probe, a radiofrequency ablation probe.

**In** some embodiments, the distal portion of the optical scope comprises a pressure sensor to gauge pressure inside the abdomen during and after insufflation.

In some embodiments, the optical port is made up of polyurethanes, Acrylonitrile Butadiene Styrene (ABS), or steel.

In some embodiments, the manual insufflator uses atmospheric air for insufflation.

In some embodiments, the peritoneoscopy port is fixed in the abdominal cavity of the patient to conduct post-operative inspections of the peritoneal cavity.

In some embodiments, the peritoneoscopy port includes a port balloon at distal end and an inflation port at proximal end. The port balloon is inflated through the inflation port with a syringe after insertion.

In some embodiments, the portable and disposable MicroPeritoneoscope enables visualization of the peritoneal cavity with entry from the abdominal wall under visual guidance under local anesthesia, or local anesthesia with sedation.

In another aspect, a disposable kit for direct visualization of the peritoneal cavity by entering from the abdominal wall of a patient with visual guidance under local anesthesia at the point of care is provided. The disposable kit includes an optical scope comprising a proximal portion and a distal portion. The proximal portion is attached to a handpiece facilitating advancement and retraction of the optical scope. The distal portion comprises a camera, and at least one light source. The distal portion of the optical scope is flexible forming a flexible distal portion with a radius of curvature ranging from 0 degrees to 140 degrees. The distal portion of the optical scope is controlled by manipulators for visualization the peritoneal cavity from multiple angles. A tubular optical trocar comprising an optical port inner sheath and a peritoneoscopy port. The optical port inner sheath comprises an opening configured to receive the optical scope and a glass tip to separate muscle layers in the peritoneal cavity under vision in the abdominal wall. The peritoneoscopy port comprises an opening to receive the optical port inner sheath and an eye to fix with skin of the patient. The optical port inner sheath is configured to pass through the peritoneoscopy port. A manual insufflator bulb for insufflation of atmospheric air with a one-way valve to expand the space within the peritoneal cavity facilitates the optical scope to move within the peritoneal cavity for visualization. The manual insufflator bulb is connected to the peritoneoscopy port and a digital display is connected to the camera of the optical scope for visualizing the peritoneal cavity. The digital display is attached to the handpiece.

In some embodiments, the peritoneoscopy port is fixed in the abdominal cavity of the patient to conduct post-operative inspections of the peritoneal cavity.

In some embodiments, the peritoneoscopy port includes a port balloon at distal end and an inflation port at proximal end. The port balloon is inflated through the inflation port with a syringe after insertion.

In some embodiments, the optical scope is of a length that ranges from 10 cm to 45 cm and a breadth that ranges from 1 mm to 10 mm.

The portable and disposable MicroPeritoneoscope provides a safe abdominal wall entry into the peritoneal cavity that requires the use of only local anesthesia or reginal anesthesia with sedation in a subset of patients requiring either a limited visualization or a repeated visualization of the peritoneal cavity. The portable and disposable MicroPeritoneoscope enhances the value of care & better clinical outcomes provided to a patient by allowing the optical scope to be used at Point-of-Care (POC) with little to no trauma or discomfort to the patient. The design is also telemedicine friendly allowing expert physicians to guide/assist relatively inexperienced physicians located in rural or community settings. The portable and disposable MicroPeritoneoscope includes a simple design and provides access to the peritoneal cavity with the smallest possible incision. The portable and disposable MicroPeritoneoscope includes a low-cost design to minimize the subsequent need for sterilization, maintenance, and repair. The portable and disposable MicroPeritoneoscope enables to perform a repeated visual examination of the peritoneal cavity at desired intervals at Point-of-Care (POC) with a port temporarily placed in the abdominal cavity. The portable and disposable MicroPeritoneoscope provides a real-time view of the intra-peritoneal structures and can appreciate the color and texture of the organs. Further, differentiation between pus and blood, pick-up of micro diseases like peritoneal secondaries, tuberculosis abdomen, and early anastomotic leak identification is facilitated.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF THE DRAWINGS

The embodiments herein will be better understood from the following detailed description with reference to the drawings, in which:
FIG. 1 illustrates a portable and disposable MicroPeritoneoscope for direct visualization of the peritoneal cavity by entering from abdominal wall of a patient with visual guidance under local anesthesia at point of care according to some embodiments herein;
FIG. 2 illustrates the distal portion of the optical scope of FIG.1 according to some embodiments herein;
FIG. 3 illustrates a structural diagram depicting a flexible distal portion of the optical scope of FIG.1 according to some embodiments herein;
FIG. 4 illustrates an inflatable balloon system placed around the optical scope of FIG.1 according to some embodiments herein;
FIG. 5 illustrates a structural diagram of a tip balloon provided at distal end of the optical scope and an inflation valve provided on the handpiece of the portable and disposable MicroPeritoneoscope of FIG.1 according to some embodiments herein;
FIG. 6 illustrates a structural diagram of an inflatable balloon system provided at a mid-portion of the optical scope with a flexible distal portion of FIG.2 according to some embodiments herein;
FIG. 7 illustrates a structural diagram of a flexible tip balloon provided at the flexible distal portion of the optical scope of FIG. 2 according to some embodiments herein;
FIG. 8 illustrates a peritoneoscopy port to conduct post-operative inspections of the peritoneal cavity according to some embodiments herein;
FIG. 9 illustrates a peritoneoscopy port with a port balloon to conduct post-operative inspections of the peritoneal cavity according to some embodiments herein; and
FIG. 10 illustrates a disposable kit for direct visualization of the peritoneal cavity by entering from the abdominal wall of a patient with visual guidance under local anesthesia at the point of care.

### DETAILED DESCRIPTION OF PREFERRED EMBODIMENTS

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

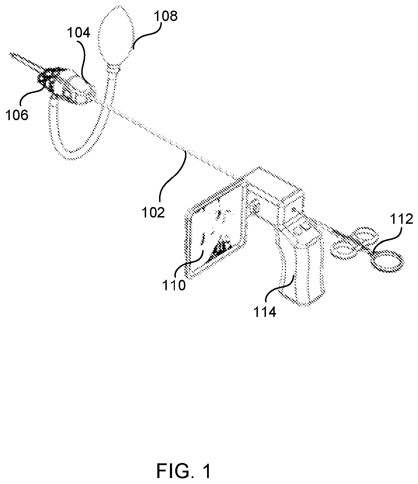

As mentioned, there remains a need for a portable & disposable MicroPeritoneoscope for direct visualization of the peritoneal cavity at point-of-care. Referring now to the drawings, and more particularly to FIGS. 1 through 10, where similar reference characters denote corresponding features consistently throughout the figures, preferred embodiments are shown.

FIG. 1 illustrates a portable and disposable MicroPeritoneoscope 100 for direct visualization of the peritoneal cavity by entering from the abdominal wall of a patient with visual guidance under local anesthesia at the point of care according to some embodiments herein. The portable and disposable MicroPeritoneoscope 100 includes an optical scope 102, a tubular trocar including an optical port inner sheath 104, and a peritoneoscopy port 106, a manual insufflator bulb 108 for insufflation of atmospheric air with a one-way valve, a digital display 110, a biopsy instrument 112, and a handpiece 114. The optical scope 102 comprises a proximal portion and a distal portion. The proximal portion of the optical scope 102 is attached to the handpiece 114 facilitating advancement and retraction of the optical scope 102. The proximal portion of the optical scope 102 includes a biopsy instrument entry port to provide access into the peritoneal cavity and the distal portion includes a camera, a biopsy exit port, and at least one light source. The optical port inner sheath 104 of the tubular optical trocar includes an opening configured to receive the optical scope 102 and a glass tip to separate muscle layers under visual guidance in the abdominal wall. The peritoneoscopy port 106 of the tubular trocar includes an opening to receive the optical port inner sheath 104 and an eye to fix with the skin of the patient. The optical port inner sheath 104 is configured to pass through the peritoneoscopy port 106. The manual insufflator bulb 108 for insufflation of atmospheric air with a one-way valve is used to expand the space within the peritoneal cavity facilitating the optical scope 102 to move within the peritoneal cavity for visualization. The manual insufflator bulb 108 is connected to the peritoneoscopy port 106. The digital display 110 is connected to the camera of the optical scope 102 for visualizing the peritoneal cavity. At least one light source provides illumination through the optical scope 102. The digital display 110 is permanently or temporarily attached to the handpiece 114. The biopsy instrument 112 is introduced through the biopsy instrument entry port for obtaining a biopsy sample from intraperitoneal organs. The distal end of the biopsy instrument 112 passes through the biopsy exit port of the optical scope 102 to access organs or soft tissue within the peritoneal cavity for obtaining the biopsy sample. In some embodiments, the digital display has at least one degree of freedom to allow physicians to adjust the view of intraperitoneal structures. The display may include two degrees of freedom. The portable and disposable MicroPeritoneoscope 100 can provide live video feed to the digital display 110. In some embodiments, buttons are provided on the handpiece 114 at the proximal end that facilitates capturing of video clips or 2D images. The portable and disposable MicroPeritoneoscope 100 includes a memory to store the video clips or 2D images obtained by the camera and a processor to process the video clips or 2D images to display on the digital display 110.

The biopsy instrument 112 passes through the proximal portion of the optical scope 102 from an insertion port provided in the handpiece 114. The optical scope 102 is of a length that ranges from 10 cm to 45 cm that facilitates reaching the pelvis from Palmer's point and breadth that ranges from 1 mm to 10 mm which is adequate to minimize pain while entering through the abdominal wall corresponding to the abdominal cavity and minimize trauma to the tissue. In some embodiments, the breadth of the optical scope 102 ranges from 2 mm to 5 mm. The portable and disposable MicroPeritoneoscope 100 enables visualization of the peritoneal cavity with entry from the abdominal wall under visual guidance under local anesthesia, regional anesthesia, sedation, or general anesthesia in some instances where local anesthesia is not feasible.

The distal portion of the optical scope 102 is flexible with a radius of curvature ranging from 0 degrees to 140 degrees. The distal portion of the optical scope 102 is controlled by manipulators for better visualization of the peritoneal cavity in multiple angles. The flexible distal portion is capable of bending in an upward and downward direction using a wire-based actuator. The distal end of the wire is hooked at the flexible distal portion of the optical scope 102 and the proximal end of the wire is looped around a dial of the manipulators provided on the handpiece 114 enabling the flexible distal portion to change angles when the dial is turned. At least one light source provides illumination through the optical scope 102 even after the bending is triggered. In some embodiments, the optical scope 102 has a flexible distal portion and a long rigid proximal portion which allows maneuverability using the long rigid proximal portion of the optical scope 102 while the flexible distal portion allows for increased range of motion. In some embodiments, the distal portion of the optical scope 102 includes a pressure sensor to measure the pressure inside the peritoneal cavity after insufflation so that appropriate pressure is maintained to avoid discomfort to the patient. In some embodiments, the pressure sensor may be provided at the distal tip of the optical scope 102. The pressure measurements obtained by the sensor may be displayed on the digital display 110, thus providing control to avoid inflation and discomfort caused to the patient. In some embodiments, a probe is inserted through the biopsy instrument entry port of the optical scope to assess the density and vascularity of intra-peritoneal structures. The probe may be used to cauterize a blood vessel to stop internal bleeding, visualize blood flow in blood vessels, etc. The probe helps to examine the local spread of pathologies in close proximity as external ultrasound examination has limited penetration. The probe is selected from an ultrasound probe, a laser probe, a radiofrequency ablation probe. In some embodiments, an additional light source is inserted through the biopsy instrument entry port of the optical scope for additional illumination.

The tubular optical trocar including the optical port inner sheath 104 and the peritoneoscopy port 106 provides safe entry into the peritoneal cavity under the visual guidance of the camera of the optical scope 102 through the digital display 110. The peritoneoscopy port 106 may be made up of polyurethanes, Acrylonitrile Butadiene Styrene, or steel. In some embodiments, the peritoneoscopy port is fixed in the abdominal cavity of the patient to conduct post-operative inspections of the peritoneal cavity using the portable and disposable MicroPeritoneoscope 100. The portable and disposable MicroPeritoneoscope 100 includes a battery system to power the instrument. The camera includes a CMOS image sensor. In some embodiments, the camera data may be communicated over a network to support telemedicine. In some embodiments, the camera data is analyzed using an Artificial Intelligence module to identify different pathologies like differences between peritoneal tuberculosis and metastatic peritoneal deposits. The Artificial Intelligence module may record changes in vascularity, size, and color which can be used as a reference point for repeated or sequential monitoring of response to chemotherapy and radiotherapy.

In some embodiments, an injection tip is provided in the distal portion of the optical scope 102 to deliver local chemotherapy for malignant tumors as this would avoid the toxicity of chemotherapy for the entire body. In some embodiments, a laser probe is provided in the distal portion of the optical scope 102 to treat different diseases like endometriosis.

In some embodiments, the distal portion of the optical scope 102 includes an inflatable tip to simultaneously manipulate different organs without injuring them to visualize the space created by the balloon at the same time, so that the organs during manipulation do not obscure the view. The inflatable tip may be interchangeable.

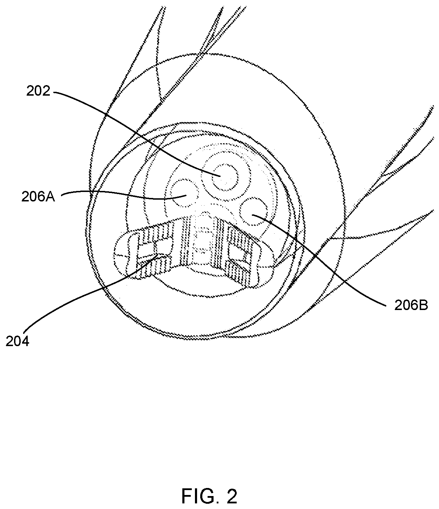

FIG. 2 illustrates the distal portion 200 of the optical scope 102 of FIG.1 according to some embodiments herein. The distal portion of the optical scope 102 includes a camera 202, a biopsy exit port 204, and at least one light source 206A-B. At least one light source 206A-B provides illumination through the optical scope 102. The camera 202 helps to visualize intra-peritoneal structures on the digital display 110. An option is provided on the digital display 110 to zoom in and visualize the intra-peritoneal structures. The biopsy instrument 112 is introduced through the biopsy instrument entry port for obtaining a biopsy sample from intraperitoneal structures. The distal end of the biopsy instrument 112 passes through the biopsy exit port 204 of the optical scope 102 to access organs or soft tissue within the peritoneal cavity for obtaining the biopsy sample.

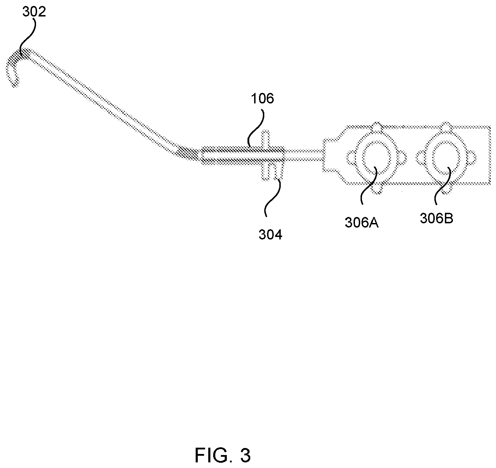

FIG. 3 illustrates a structural diagram 300 depicting a flexible distal portion of the optical scope 102 of FIG.1 according to some embodiments herein. Diagram 300 shows the flexible distal portion 302 of the optical scope 102, the peritoneoscopy port 106, a connection port 304 for the manual insufflator bulb 108, and tip manipulators 306A-B. The flexible distal portion 302 of the optical scope 102 has a radius of curvature ranging from 0 degrees to 140 degrees. The flexible distal portion 302 of the optical scope 102 is controlled by the manipulators 306A-B for better visualization of the peritoneal cavity in multiple angles. The flexible distal portion 302 is capable of bending in an upward and downward direction using a wire-based actuator. The distal end of the wire is hooked at the flexible distal portion 302 of the optical scope 102 and the proximal end of the wire is looped around a dial of the manipulators 306A-B provided on the handpiece 114 enabling the flexible distal portion 302 to change angles when the dial is turned. The flexible distal portion 302 is capable of reaching the pelvis and posterior surface of the liver. At least one light source provides illumination through the optical scope 102 even after the bending is triggered. In an embodiment, a port with two-point angulation is provided. A semi-rigid hollow sheath is introduced into the abdominal cavity using a metallic sharp trocar after which the trocar is removed and the semi-rigid sheath is left in place.

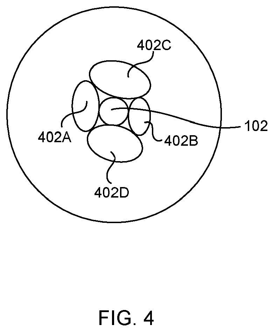

FIG. 4 illustrates an inflatable balloon system 400 placed around the optical scope 102 of FIG.1 according to an example not covered by the claims. The inflatable balloon system 400 comprises four compartments 402A-D, which can be inflated using a valve mechanism and an inflation system. Each compartment inflates into a high-volume low-pressure balloon that will aid in the navigation of the optical scope 102. For example, if the user wants the optical scope 102 to turn to the right, the balloon on the left 402A can be inflated deflecting the optical scope 102 to the right.

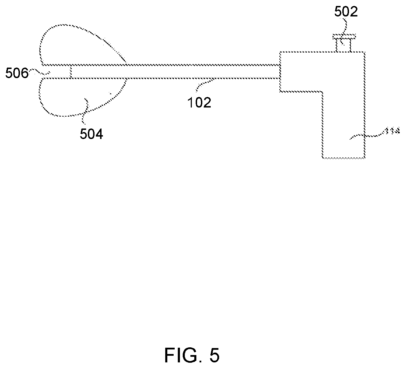

FIG. 5 illustrates a structural diagram 500 of a tip balloon at provided at distal end of the optical scope and an inflation valve provided on the handpiece of the portable and disposable MicroPeritoneoscope of FIG.1 according to an example not covered by the claims. Diagram 500 shows the inflation valve 502, the tip balloon 504, a space for visualization 506, the optical scope 102, and the handpiece 114. The tip balloon 504 can be inflated with the inflation valve 502 provided on the handpiece 114 when there is a need to manipulate and visualize simultaneously without injuring nearby organs for example lower surface of the liver or in-between areas of the intestine. The tip balloon 504 can be inflated when there is a need to manipulate and visualize beneath the organs like the lower surface of the liver or in-between areas of the intestine. The tip balloon 504 will be helpful when examining the bleeding edges of the intestine post operatively or to access the local spread of tumors and also to visualize the space in-between the intestine for lymphomas, aneurysms and perforation by manipulating the intestine.

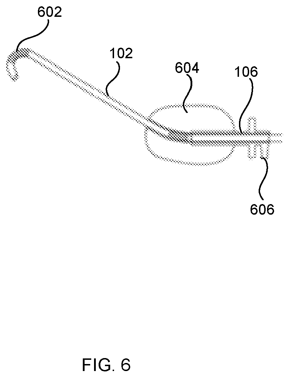

FIG. 6 illustrates a structural diagram 600 of an inflatable balloon system provided at a mid-portion of the optical scope 102 with a flexible distal portion of FIG.2 according to an example not covered by the claims. Diagram 600 shows the flexible distal portion 602 of the optical scope 102, the inflatable balloon system 604, the peritoneoscopy port 106, and a connection port 604 for the manual insufflator bulb 108. The inflatable balloon system 604 can be inflated with an inflation valve from the handpiece 114. The inflatable balloon system 604 provides stability and maneuverability of the optical scope 102.

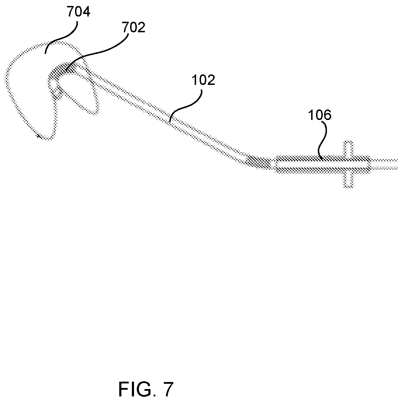

FIG. 7 illustrates a structural diagram 700 of a flexible tip balloon provided at the flexible distal portion of the optical scope of FIG. 2 according to some embodiments herein; Diagram 700 shows the flexible distal portion 702, the flexible tip balloon 704, and the peritoneoscopy port 106. The flexible tip balloon 704 can be inflated with an inflation valve from the handpiece 114. The flexible tip balloon 704 can be inflated when there is a need to manipulate and visualize beneath the organs like the lower surface of the liver or in-between areas of the intestine. The flexible tip balloon 704 will be helpful when examining the bleeding edges of the intestine post operatives or to access the local spread of tumors and also to visualize the space in-between the intestine for lymphomas, aneurysms and perforation by mutilating the intestine.

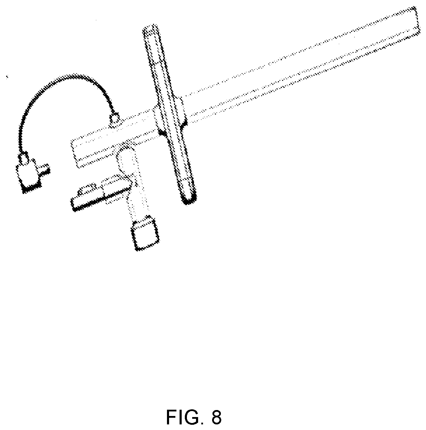

FIG. 8 illustrates a peritoneoscopy port 800 to conduct post-operative inspections of the peritoneal cavity according to some embodiments herein. The peritoneoscopy port 800 is fixed over the abdominal wall of the patient for repeated or sequential examination using the portable and disposable MicroPeritoneoscope 100.

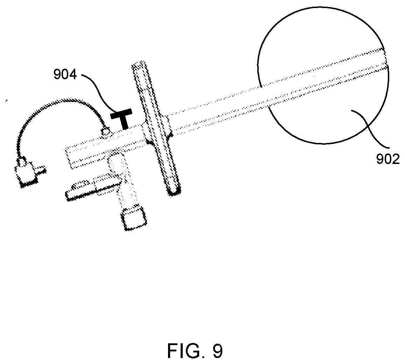

FIG. 9 illustrates a peritoneoscopy port 900 with a port balloon to conduct post-operative inspections of the peritoneal cavity according to some embodiments herein. The peritoneoscopy port 900 includes the port balloon 902 at the distal end and an inflation port 904 to inflate the port balloon 902 at the proximal end. The port balloon 902 is provided at the distal end of the peritoneoscopy port 900. The port balloon 902 prevents the peritoneoscopy port 900 from coming out and will hold the peritoneoscopy port 900 in place and the compliant and spherical soft tip will prevent any injury to the internal organs and any discomfort to the patient as it will be left behind for days for sequential monitoring. The port balloon 902 can be inflated through the inflation port 904 with a syringe after insertion.

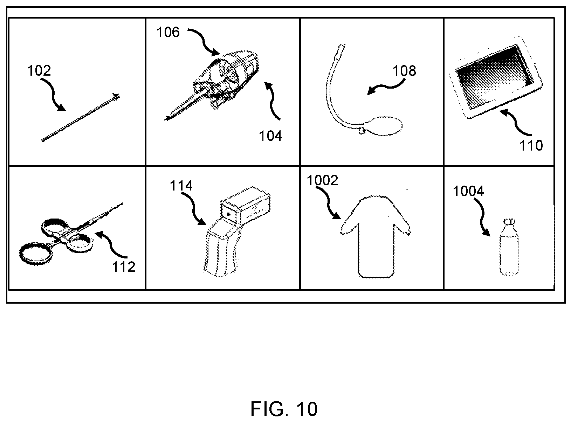

FIG. 10 illustrates a disposable kit 1000 for direct visualization of the peritoneal cavity by entering from the abdominal wall of a patient with visual guidance under local anesthesia at the point of care. The disposable kit 1000 includes an optical scope 102, a tubular optical trocar comprising an optical port inner sheath 104 and a peritoneoscopy port 106, a manual insufflator bulb 108 for insufflation of atmospheric air, a digital display 110, a biopsy instrument 112, a handpiece 114, a surgical drape 1002 and a local anesthesia 1004. The functioning of the parts has been described above.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modifications.

## Claims

1. A portable and disposable MicroPeritoneoscope (100) for direct visualization of peritoneal cavity by entering from abdominal wall of a patient with visual guidance under local anesthesia at point of care, wherein the portable and disposable MicroPeritoneoscope (100) comprises:
an optical scope (102) comprising a proximal portion and a distal portion, wherein the proximal portion is attached to a handpiece facilitating advancement and retraction of the optical scope (102), wherein the proximal portion comprises a biopsy instrument entry port to provide access into the peritoneal cavity, wherein the distal portion comprises a camera (202), a biopsy exit port (204), and at least one light source (206A-B), wherein the distal portion of the optical scope (102) is flexible forming a flexible distal portion (302, 602, 702) with a radius of curvature ranging from 0 degrees to 140 degrees, wherein the distal portion of the optical scope (102) is controlled by manipulators (306A-B) for visualization the peritoneal cavity from multiple angles;
a tubular optical trocar comprising an optical port inner sheath (104) and an peritoneoscopy port (106, 800, 900), wherein the optical port inner sheath (104) comprises an opening configured to receive the optical scope (102) and a glass tip to separate muscle layers under vision in the abdominal wall, wherein the peritoneoscopy port (106, 800, 900) comprises an opening to receive the optical port inner sheath (104) and an eye to fix with skin of the patient, wherein the optical port inner sheath (104) is configured to pass through the peritoneoscopy port (106, 800, 900) and fixed inside the peritoneoscopy port (106, 800, 900);
a manual insufflator bulb (108) for insufflation of atmospheric air with one-way valve to expand the space within the peritoneal cavity facilitating the optical scope (102) to move within the peritoneal cavity for visualization, wherein the manual insufflator bulb (108) is connected to the peritoneoscopy port (106, 800, 900);
a digital display (110) that is connected to the camera (202) of the optical scope (102) for visualizing the peritoneal cavity, wherein the digital display (110) is attached to the handpiece (114); and
a biopsy instrument (112) that is introduced through the biopsy instrument entry port for obtaining a biopsy sample from intraperitoneal structures, wherein distal end of the biopsy instrument (112) passes through the biopsy exit port (204) of the optical scope (102) to access organs or soft tissue within the peritoneal cavity for obtaining the biopsy sample.

2. The portable and disposable MicroPeritoneoscope (100) as claimed in claim 1, wherein the optical scope (102) is of a length that ranges from 10 cm to 45 cm and a breadth that ranges from 1 mm to 10 mm.

3. The portable and disposable MicroPeritoneoscope (100) as claimed in claim 1, wherein a probe is inserted through the biopsy instrument entry port of the optical scope (102) to assess the density and vascularity of intra-peritoneal structures or to ablate and cauterize a blood vessel, wherein the probe is selected from an ultrasound probe, a laser probe, a radiofrequency ablation probe.

4. The portable and disposable MicroPeritoneoscope (100) as claimed in claim 1, wherein the distal portion of the optical scope (102) comprises a pressure sensor to gauge pressure inside the abdomen during and after insufflation.

5. The portable and disposable MicroPeritoneoscope (100) as claimed in claim 1, wherein the optical port inner sheath (104) and peritoneoscopy port (106, 800, 900) is made up of polyurethanes, Acrylonitrile Butadiene Styrene (ABS), or steel.

6. The portable and disposable MicroPeritoneoscope (100) as claimed in claim 1, wherein the peritoneoscopy port (106, 800, 900) is fixed in the abdominal cavity of the patient to conduct post-operative inspections of the peritoneal cavity.

7. The portable and disposable MicroPeritoneoscope (100) as claimed in claim 1, wherein the peritoneoscopy port (106, 800, 900) comprises a port balloon (902) at distal end and an inflation port (904) at proximal end, wherein the port balloon (902) is inflated through the inflation port (904) with a syringe after insertion.

8. The portable and disposable MicroPeritoneoscope (100) as claimed in claim 1, wherein the MicroPeritoneoscope (100) enables visualization of the peritoneal cavity with entry from the abdominal wall under visual guidance under local anesthesia, reginal anesthesia, sedation, or general anesthesia.

9. A disposable kit (1000) comprising a portable and disposable MicroPeritoneouscope (100) as claimed in claim 1, a surgical drape (1002); and a local anesthesia (1004).

10. The disposable kit (1000) as claimed in claim 9, wherein the peritoneoscopy port (106, 800, 900) is fixed in the abdominal cavity of the patient to conduct post-operative inspections of the peritoneal cavity, wherein the peritoneoscopy port (106, 800, 900) comprises a port balloon (902) at distal end and an inflation port (904) at proximal end, wherein the port balloon (902) is inflated through the inflation port (904) with a syringe after insertion.

11. The disposable kit (1000) as claimed in claim 9, wherein the optical scope (102) is of a length that ranges from 10 cm to 45 cm and a breadth that ranges from 1 mm to 10 mm.

## Patentansprüche

1. Tragbares und wegwerfbares Mikroperitoneoskop (100) zur direkten Visualisierung der Peritonealhöhle durch Eintritt über die Bauchdecke eines Patienten mit visueller Führung unter lokaler Anästhesie am Ort der Behandlung, wobei das tragbare und wegwerfbare Mikroperitoneoskop (100) umfasst:
ein optisches Endoskop (102), das einen proximalen Abschnitt und einen distalen Abschnitt umfasst, wobei der proximale Abschnitt an einem Handstück befestigt ist, das das Vorschieben und Zurückziehen des optischen Endoskops (102) erleichtert, wobei der proximale Abschnitt eine Einführungsöffnung für ein Biopsieinstrument umfasst, um einen Zugang in die Peritonealhöhle zu schaffen, wobei der distale Abschnitt eine Kamera (202), eine Biopsie-Ausgangsöffnung (204) und mindestens eine Lichtquelle (206A-B) umfasst, wobei der distale Abschnitt des optischen Endoskops (102) flexibel ist und einen flexiblen distalen Abschnitt (302, 602, 702) mit einem Krümmungsradius im Bereich von 0 Grad bis 140 Grad bildet, wobei der distale Abschnitt des optischen Endoskops (102) durch Manipulatoren (306A-B) zur Visualisierung der Peritonealhöhle aus mehreren Winkeln gesteuert wird;
einen röhrenförmigen optischen Trokar, der eine innere Hülle (104) der optischen Öffnung und eine Peritoneoskopie-Öffnung (106, 800, 900) umfasst, wobei die innere Hülle (104) der optischen Öffnung eine Öffnung umfasst, die so konfiguriert ist, dass sie das optische Endoskop (102) und eine Glasspitze aufnimmt, um Muskelschichten unter Sicht in der Bauchdecke zu trennen, wobei die Peritoneoskopie-Öffnung (106, 800, 900) eine Öffnung zur Aufnahme der inneren Hülle der optischen Öffnung (104) und eine Kamera zur Fixierung an der Haut des Patienten umfasst, wobei die innere Hülle der optischen Öffnung (104) so konfiguriert ist, dass sie durch die Peritoneoskopie-Öffnung (106, 800, 900) hindurchgeht und innerhalb der Peritoneoskopie-Öffnung (106, 800, 900) fixiert ist;
ein manueller Insufflatorballon (108) zur Insufflation von atmosphärischer Luft mit einem Einwegventil, um den Raum innerhalb der Peritonealhöhle zu erweitern, was es dem optischen Endoskop (102) erleichtert, sich innerhalb der Peritonealhöhle zur Visualisierung zu bewegen, wobei der manueller Insufflatorballon (108) mit der Peritoneoskopie-Öffnung (106, 800, 900) verbunden ist;
eine digitale Anzeige (110), die mit der Kamera (202) des optischen Endoskops (102) zur Visualisierung der Peritonealhöhle verbunden ist, wobei die digitale Anzeige (110) an dem Handstück (114) angebracht ist; und
ein Biopsieinstrument (112), das durch die Einführungsöffnung für ein Biopsieinstrument eingeführt wird, um eine Biopsieprobe von intraperitonealen Strukturen zu erhalten, wobei das distale Ende des Biopsieinstruments (112) durch die Biopsie-Ausgangsöffnung (204) des optischen Endoskops (102) führt, um Zugang zu Organen oder Weichgewebe in der Peritonealhöhle für die Entnahme der Biopsieprobe zu erhalten.

2. Tragbares und wegwerfbares Mikroperitoneoskop (100) nach Anspruch 1, wobei das optische Endoskop (102) eine Länge im Bereich von 10 cm bis 45 cm und eine Breite im Bereich von 1 mm bis 10 mm aufweist.

3. Tragbares und wegwerfbares Mikroperitoneoskop (100) nach Anspruch 1, wobei eine Sonde durch die Einführungsöffnung für das Biopsieinstrument des optischen Endoskops (102) eingeführt wird, um die Dichte und Vaskularität von intraperitonealen Strukturen zu beurteilen oder ein Blutgefäß abzutragen und zu kauterisieren, wobei die Sonde aus einer Ultraschallsonde, einer Lasersonde, einer Radiofrequenzablationssonde ausgewählt ist.

4. Tragbares und wegwerfbares Mikroperitoneoskop (100) nach Anspruch 1, wobei der distale Abschnitt des optischen Endoskops (102) einen Drucksensor zur Messung des Drucks innerhalb des Abdomens während und nach der Insufflation umfasst.

5. Tragbares und wegwerfbares Mikroperitoneoskop (100) nach Anspruch 1, wobei die innere Hülle der optischen Öffnung (104) und die Peritoneoskopie-Öffnung (106, 800, 900) aus Polyurethane, Acrylnitril-Butadien-Styrol (ABS) oder Stahl bestehen.

6. Tragbares und wegwerfbares Mikroperitoneoskop (100) nach Anspruch 1, wobei die Peritoneoskopie-Öffnung (106, 800, 900) in der Bauchhöhle des Patienten befestigt ist, um postoperative Inspektionen der Peritonealhöhle durchzuführen.

7. Tragbares und wegwerfbares Mikroperitoneoskop (100) nach Anspruch 1, wobei die Peritoneoskopie-Öffnung (106, 800, 900) einen Öffnungsballon (902) am distalen Ende und eine Aufblasöffnung (904) am proximalen Ende umfasst, wobei der Öffnungsballon (902) nach dem Einführen durch die Aufblasöffnung (904) mit einer Spritze inflatiert wird.

8. Tragbares und wegwerfbares Mikroperitoneoskop (100) nach Anspruch 1, wobei das Mikroperitoneoskop (100) die Visualisierung der Peritonealhöhle mit Zugang von der Bauchdecke aus unter visueller Führung unter Lokalanästhesie, Regionalanästhesie, Sedierung oder Vollnarkose ermöglicht.

9. Einwegkit (1000), umfassend ein tragbares und wegwerfbares Mikroperitoneoskop (100) nach Anspruch 1, ein chirurgisches Tuch (1002) und ein Lokalanästhetikum (1004).

10. Einwegkit (1000) nach Anspruch 9, wobei die Peritoneoskopie-Öffnung (106, 800, 900) in der Bauchhöhle des Patienten befestigt ist, um postoperative Inspektionen der Peritonealhöhle durchzuführen, wobei die Peritoneoskopie-Öffnung (106, 800, 900) einen Öffnungsballon (902) am distalen Ende und eine Aufblasöffnung (904) am proximalen Ende aufweist, wobei der Öffnungsballon (902) nach dem Einführen durch die Aufblasöffnung (904) mit einer Spritze inflatiert wird.

11. Einwegkit (1000) nach Anspruch 9, wobei das optische Endoskop (102) eine Länge im Bereich von 10 cm bis 45 cm und eine Breite im Bereich von 1 mm bis 10 mm aufweist.

## Revendications

1. Micropéritonéoscope portable et jetable (100) pour la visualisation directe de la cavité péritonéale en pénétrant à partir de la paroi abdominale d'un patient avec un guidage visuel sous anesthésie locale au point de soins, dans lequel le micropéritonéoscope portable et jetable (100) comprend :
un dispositif d'observation optique (102) comprenant une partie proximale et une partie distale, la partie proximale étant fixée à une pièce à main facilitant l'avancement et la rétraction du dispositif d'observation optique (102), la partie proximale comprenant un orifice d'entrée de l'instrument de biopsie pour permettre l'accès à l'intérieur de la cavité péritonéale, la partie distale comprenant une caméra (202), un orifice de sortie de biopsie (204), et au moins une source de lumière (206A-B), la partie distale du dispositif d'observation optique (102) étant flexible, formant une partie distale flexible (302, 602, 702) avec un rayon de courbure allant de 0 degré à 140 degrés, la partie distale du dispositif d'observation optique (102) étant contrôlée par des manipulateurs (306A-B) pour la visualisation de la cavité péritonéale sous plusieurs angles ;
un trocart optique tubulaire comprenant une gaine interne de l'orifice optique (104) et un orifice de péritonéoscopie (106, 800, 900), la gaine interne de l'orifice optique (104) comprenant une ouverture configurée pour recevoir le dispositif d'observation optique (102) et une pointe en verre pour séparer les couches musculaires sous vision dans la paroi abdominale, l'orifice de péritonéoscopie (106, 800, 900) comprenant une ouverture pour recevoir la gaine intérieure de l'orifice optique (104) et une caméra pour fixer à la peau du patient, la gaine intérieure de l'orifice optique (104) étant configurée pour passer à travers l'orifice de péritonéoscopie (106, 800, 900) et fixée à l'intérieur de l'orifice de péritonéoscopie (106, 800, 900) ;
une ampoule d'insufflateur manuelle (108) pour l'insufflation d'air atmosphérique avec une valve unidirectionnelle pour élargir l'espace à l'intérieur de la cavité péritonéale facilitant le déplacement du dispositif d'observation optique (102) à l'intérieur de la cavité péritonéale pour la visualisation, l'ampoule d'insufflateur manuelle (108) étant connectée à l'orifice de péritonéoscopie (106, 800, 900) ;
un écran numérique (110) qui est connecté à la caméra (202) du dispositif d'observation optique (102) pour visualiser la cavité péritonéale, l'écran numérique (110) étant fixé à la pièce à main (114) ; et
un instrument de biopsie (112) qui est introduit à travers l'orifice d'entrée de l'instrument de biopsie pour obtenir un échantillon de biopsie des structures intrapéritonéales, l'extrémité distale de l'instrument de biopsie (112) passant par l'orifice de sortie de biopsie (204) du dispositif d'observation optique (102) pour accéder aux organes ou aux tissus mous à l'intérieur de la cavité péritonéale afin d'obtenir l'échantillon de biopsie.

2. Micropéritonéoscope portable et jetable (100) selon la revendication 1, dans lequel le dispositif d'observation optique (102) a une longueur comprise entre 10 cm et 45 cm et une largeur comprise entre 1 mm et 10 mm.

3. Micropéritonéoscope portable et jetable (100) selon la revendication 1, dans lequel une sonde est insérée à travers l'orifice d'entrée de l'instrument de biopsie du dispositif d'observation optique (102) pour évaluer la densité et la vascularisation des structures intrapéritonéales ou pour ablater et cautériser un vaisseau sanguin, la sonde étant choisie parmi une sonde à ultrasons, une sonde laser, une sonde d'ablation par radiofréquence.

4. Micropéritonéoscope portable et jetable (100) selon la revendication 1, dans lequel la partie distale du dispositif d'observation optique (102) comprend un capteur de pression pour mesurer la pression à l'intérieur de l'abdomen pendant et après l'insufflation.

5. Micropéritonéoscope portable et jetable (100) selon la revendication 1, dans lequel la gaine interne de l'orifice optique (104) et l'orifice de péritonéoscopie (106, 800, 900) sont constitués de polyuréthanes, d'Acrylonitrile Butadiène Styrène (ABS), ou d'acier.

6. Micropéritonéoscope portable et jetable (100) selon la revendication 1, dans lequel l'orifice de péritonéoscopie (106, 800, 900) est fixé dans la cavité 2 abdominale du patient pour effectuer des inspections post-opératoires de la cavité péritonéale.

7. Micropéritonéoscope portable et jetable (100) selon la revendication 1, dans lequel l'orifice de péritonéoscopie (106, 800, 900) comprend un ballon d'orifice (902) à l'extrémité distale et un orifice d'inflation (904) à l'extrémité proximale, le ballon d'orifice (902) étant gonflé à travers l'orifice d'inflation (904) à l'aide d'une seringue après l'insertion.

8. Micropéritonéoscope portable et jetable (100) selon la revendication 1, dans lequel le micropéritonéoscope (100) permet la visualisation de la cavité péritonéale avec une entrée à partir de la paroi abdominale sous guidage visuel sous anesthésie locale, anesthésie régionale, sédation, ou anesthésie générale.

9. Kit jetable (1000) comprenant un micropéritonéoscope portable et jetable (100) selon la revendication 1, un drap chirurgical (1002) ; et une anesthésie locale (1004).

10. Kit jetable (1000) selon la revendication 9, dans lequel l'orifice de péritonéoscopie (106, 800, 900) est fixé dans la cavité abdominale du patient pour effectuer des inspections post-opératoires de la cavité péritonéale, l'orifice de péritonéoscopie (106, 800, 900) comprenant un ballon d'orifice (902) à l'extrémité distale et un orifice d'inflation (904) à l'extrémité proximale, le ballon d'orifice (902) étant gonflé à travers l'orifice d'inflation (904) à l'aide d'une seringue après l'insertion.

11. Kit jetable (1000) selon la revendication 9, dans lequel le dispositif d'observation optique (102) a une longueur comprise entre 10 cm et 45 cm et une largeur comprise entre 1 mm et 10 mm.
